(19) Europäisches Patentamt
European Patent Office
Office européen des brevets

(11) Veröffentlichungsnummer : **0 011 855**
**B1**

(12) # EUROPÄISCHE PATENTSCHRIFT

(45) Veröffentlichungstag der Patentschrift : **07.04.82**

(51) Int. Cl.³ : **C 07 C 69/732**, C 07 C 67/22

(21) Anmeldenummer : **79104735.0**

(22) Anmeldetag : **28.11.79**

(54) **Verfahren zur Herstellung von Alkylestern ungesättigter alpha-Hydroxycarbonsäuren.**

(30) Priorität : **04.12.78 DE 2852343**

(43) Veröffentlichungstag der Anmeldung : **11.06.80 (Patentblatt 80/12)**

(45) Bekanntmachung des Hinweises auf die Patenterteilung : **07.04.82 Patentblatt 82/14**

(84) Benannte Vertragsstaaten : **BE CH DE FR GB NL**

(56) Entgegenhaltungen :
**CH - A5 - 586 179**
**DE - C - 562 390**
**GB - A - 264 143**
**JOURNAL OF THE CHEMICAL SOCIETY, 1956, London, Seiten 3240**
**JOURNAL OF HETEROCYCLIC CHEMISTRY, Bd. 4, 1967, J.D. EDWARDS et al. : « The Synthesis of SenecIphylic Acid (1) », Seiten 487 bis 490**
**THE JOURNAL OF ORGANIC CHEMISTRY, Bd. 37, n° 7, 1972 B.C. HARTMAN und B. RICKBORN : « The Lithium Salt catalyzed Rearrangement of Epoxides. II. Glycidic Esters », Seiten 943 bis 946**

(73) Patentinhaber : **BASF Aktiengesellschaft**
**Carl-Bosch-Strasse 38**
**D-6700 Ludwigshafen (DE)**

(72) Erfinder : **Kroener, Michael, Dr. Dipl.-Chem.**
**Eislebener Weg 8**
**D-6800 Mannheim 42 (DE)**
Erfinder : **Goetze, Walter, Dr. Dipl.-Chem.**
**Duerkheimer Strasse 13**
**D-6701 Dannstadt-Schauernheim 1 (DE)**

**EP 0 011 855 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Imprimerie Jouve, 18, rue St-Denis, 75001 Paris, France

Verfahren zur Herstellung von Alkylestern ungesättigter alpha-Hydroxycarbonsäuren

Die Erfindung betrifft ein Verfahren zur Herstellung von Alkenylmilchsäureestern und neue Alkenylmilchsäureester.

Es ist bereits bekannt, daß man Cyanhydrine mit Alkohol und Chlorwasserstoff zu Iminoesterhydrochloriden umsetzen kann, aus denen man durch Hydrolyse die entsprechenden α-Hydroxycarbonsäureester erhält. Diese von A. Pinner (« Die Iminoäther und ihre Derivate », Verlag Oppenheimer, Berlin 1892) eingeführte Umsetzung läßt sich mit sehr vielen aliphatischen und aromatischen Nitrilen durchführen. C = C-ungesättigte Nitrile, z.B. Allylcyanid (A. Pinner, Chem. Ber. *17,* 2 007 (1884)) addieren jedoch bei dieser Reaktion mehr oder weniger leicht Chlorwasserstoff an die C = C-Doppelbindung, was zu starken Ausbeuteverminderungen führt.

Die Darstellung des Methylesters der Vinylmilchsäure ist in der Literatur bereits beschrieben (J. Chem. Soc. *1956,* 3 239). Sie erfolgt in sehr umständlicher Weise :

Das Cyanhydrin aus Methylvinylketon und Blausäure wird zunächst bei 90 °C mit konzentrierter Salzsäure behandelt, die entstandene wasserlösliche Vinylmilchsäure mit Ether extrahiert und mit Diazomethan zum Methylester umgesetzt. Die Ausbeute beträgt hierbei 55 %.

Gemäß DE-OS 2 711 381 erhält man den Butylester der Vinylmilchsäure durch Umsetzen von letzterer mit Butanol in Gegenwart von Salzsäure.

Schließlich wird in J. Heteroc. Chem. *1967,* 488 die Herstellung von Isopropenylmilchsäuremethylester (Methyl-2,3-dimethyl-2-hydroxy-3-butenoat) beschrieben, welche durch Umsetzung des entsprechenden Cyanhydrins mit Methanol/HCl in Ether und anschließende Hydrolyse des Orthoesters erfolgt. Die Ausbeute beträgt bei dieser Reaktion insgesamt etwa 40 %.

Gegenstand der Erfindung ist ein Verfahren zur Herstellung von Alkenylmilchsäureestern der allgemeinen Formel I

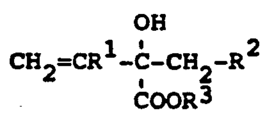

(I)

worin

$R^1$ ein Wasserstoffatom oder eine Methyl- oder Ethylgruppe

$R^2$ ein Wasserstoffatom oder eine Alkylgruppe mit 1-3 Kohlenstoffatomen und

$R^3$ eine Alkylgruppe mit 1-6 Kohlenstoffatomen

darstellen, welches darin besteht, daß man in Cyanhydrin der allgemeinen Formel II

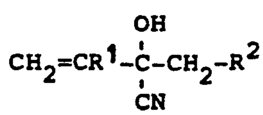

(II)

worin

$R^1$ und $R^2$ dasselbe wie oben bedeuten, mit einem Alkohol der allgemeinen Formel III

$$R^3OH \qquad (III)$$

worin

$R^3$ dasselbe wie oben bedeutet, in Gegenwart von Chlorwasserstoff umsetzt und das so erhaltene Reaktionsgemisch anschließend direkt unter Zugabe von Wasser bei ptt 1-6 hydrolysiert.

Bei der Umsetzung von II und III mit Chlorwasserstoff bilden sich die Iminoesterhydrochloride, die ohne Isolierung weiterverarbeitet werden.

Die Anlagerung von III an II erfolgt bei – 30 bis + 50 °C, vorzugsweise bei – 10 bis + 30 °C. Man kann mit stöchiometrischen Mengen Alkohol arbeiten, es empfiehlt sich jedoch — um das Reaktionsgemisch rührfähig zu halten — mit Überschüssen bis zu 10 Mol, vorzugsweise 1,1 bis 3 Mol, Alkohol pro Mol Cyanhydrin zu arbeiten. Die Umsetzung erfolgt in der Regel ohne Zusatz weiterer Lösungsmittel. Andere inerte Lösungsmittel stören die Reaktion jedoch nicht. Chlorwasserstoffgas oder flüssiger Chlorwasserstoff wird in stöchiometrischer Menge oder bis zur Sättigung in die Reaktionsmischung eingeleitet. Zweckmäßigerweise verwendet man den Chlorwasserstoff in einem Überschuß von 1,1 bis 3 Mol pro Mol Cyanhydrin.

Das für die erste Reaktionsstufe verwendete Cyanhydrin braucht nicht vollständig wasserfrei oder durch Destillation gereinigt zu sein. Am einfachsten und sichersten läßt sich eine technisch leicht zugängliche, blausäurearme Gleichgewichtsmischung (1) in der oben genannten Weise mit Chlorwasserstoff umsetzen (vgl. DE-OS 2 655 715) :

$$CH_2{=}CH{-}CO{-}CH_3 + HCN \xrightleftharpoons{-40\,^{\circ}C} CH_2{=}CH{-}\underset{CN}{\overset{OH}{C}}{-}CH_3 \quad (I)$$

Dabei werden gleichzeitig die noch im Gleichgewicht entstandenen hochtoxischen Ausgangsstoffe unschädlich gemacht.

Die in dem ersten Reaktionsschritt entstehenden Imidate werden zweckmäßigerweise direkt weiterverarbeitet. Der überschüssige Chlorwasserstoff kann abgezogen oder neutralisiert werden. Die Hydrolyse erfolgt bei pH 1-6, vorzugsweise 2-4, bei + 10 bis + 70 °C, vorzugsweise + 20 bis + 50 °C. Verwendet man dazu wäßrige Basen, findet die Hydrolyse gleichzeitig mit der Neutralisation statt. Man erhält neben einer wäßrigen Phase, die Ammoniumchlorid und ggf. Natriumchlorid enthält, eine organische Phase, die gewaschen und destillativ aufgearbeitet wird. Arbeitet man mit annähernd stöchiometrischen Wassermengen, fällt das Ammoniumchlorid in fester, filtrierbarer Form an.

Das neue Verfahren besitzt folgende Vorteile :

1. Es können technisch leicht zugängliche und leicht zu handhabende Cyanhydrin-Gleichgewichtsmischungen als Ausgangsmaterial verwendet werden.

2. Das Verfahren führt nicht über freie Alkenylmilchsäuren, deren Isolierung sehr aufwendig ist und die anschließend verestert werden müssen.

3. In der Regel kann auf den Zusatz inerter Lösungsmittel verzichtet werden. Lediglich bei den Methylestern empfiehlt es sich, die wäßrige Phase zur Ausbeutesteigerung mit einem mit Wasser nicht mischbaren Lösungsmittel, z.B. Ether, nachzuextrahieren, wenn die Aufarbeitung aus wäßriger Lösung erfolgt.

4. Das Verfahren kann als Eintopfverfahren durchgeführt werden, ist sehr umweltfreundlich, liefert wenig Nebenprodukte und ist mit einem nur geringen Sicherheitsrisiko behaftet.

5. Es kann vollkontinuierlich durchgeführt werden. Dabei lassen sich die große Lösungswärme von Chlorwasserstoff in Alkoholen (wenn gasförmiges HCl zum Einsatz kommt), die starke Exothermität der Imidatbildung, die Hydrolyse der Imidate und ggf. die Neutralisation von überschüssigem Chlorwasserstoff gut beherrschen.

Der Verlauf der neuen Reaktion ist insofern überraschend, als nicht erwartet werden konnte, daß die Reaktion praktisch ohne Nebenreaktionen, wie z.B. Anlagerung von HCl an die C-C-Doppelbindung, abläuft. Weiter war auch nicht vorhersehbar, daß bei der Umsetzung praktisch keine Säureamide gebildet werden. Die Ausbeute der Reaktion ist infolgedessen sehr hoch.

Die erfindungsgemäß hergestellten Verbindungen eignen sich zur Herstellung von Alkaloiden (J. Heteroc. Chem. *1967,* 487) von Pflanzenschutzmitteln (z.B. Vinclozolin, vergleiche DE-OS 2 207 576, Beispiel 1) und als Monomere zur Mischpoly merisation (DE-OS 1 795 312).

Die folgenden Beispiel erläutern die Erfindung weiter. Als « Teile » sind Gewichtsteile zu verstehen.

## Beispiel 1

48,5 Teile 97 %iges Vinylmilchsäurenitril werden bei einer Temperatur von 0 °C zu einer Lösung von 17,7 Teilen Chlorwasserstoff in 17,6 Teilen Methanol zugegeben. Nach ca. 2 Stunden kommt es zur spontanen Kristallisation, wobei die Temperatur auf + 10 °C ansteigt. Man läßt noch 12 Stunden bei + 5 °C stehen und hydrolysiert dann bei Raumtemperatur mit 80 Teilen Wasser. Die sich zunächst bildende klare Lösung zerfällt nach kurzer Zeit in 2 Phasen. Man trennt die organische Phase ab, extrahiert die wäßrige Phase mit Ether und destilliert die vereinigten organischen Phasen nach dem Trocknen. Man erhält 56,7 Teile Vinylmilchsäuremethylester (90 % d. Th.). Die gaschromatische Reinheit beträgt 99,7 % ; $n_D^{20}$ : 1,432 5 ; $Kp_{20}$ : 59 °C.

## Beispiel 2

48,5 Teile 97 %iges Vinylmilchsäurenitril werden zu einer Lösung von 18 Teilen Chlorwasserstoff in 37 Teilen n-BuOH bei 0 °C zugegeben. Nach ca. 1 1/2 Stunden kristallisiert die Reaktionsmasse durch. Man läßt noch 24 Stunden bei + 5 °C stehen und hydrolysiert dann bei Raumtemperatur mit 80 Teilen Wasser. Die sich zunächst bildende klare Lösung trübt sich nach kurzer Zeit. Nach 1 1/2 Stunden wird die organische Phase abgetrennt, gewaschen und destilliert. Man erhält 80,2 Teile Vinylmilchsäure-n-butylester (96 % d. Th.) vom Siedepunkt $Kp_{11}$ : 72-73 °C.

## Beispiel 3

Zu 253 Teilen Isobutanol werden bei – 15 °C gleichzeitig 56 Teile wasserfreie Blausäure (99 %ig), 140 Teile Methylvinylketon (98 %ig) und 2 Teile Triethylamin innerhalb ca. 1 Std. zugegeben. Anschließend wird die Reaktionsmischung 2 Stunden bei – 40 °C gehalten. Durch analytische Bestimmung der nicht umgesetzten Blausäure errechnet sich ein Cyanhydrinumsatz von 90,0 %. Nun wird mit 121 Teilen Chlorwasserstoff begast, wobei die Temperatur bis 0 °C, zur Nachreaktion innerhalb 11 Stunden bis auf + 15 °C ansteigt. Dabei verwandelt sich die zunächst klare Reaktionslösung langsam in eine Suspension.

Innerhalb 1 Stunde werden 520 Teile 10 %ige Natronlauge unter Rühren zugegeben. Dabei steigt der pH auf 3. Durch Kühlen hält man die Temperatur auf unter 20 °C. Man läßt noch 1 Stunde bei 30 °C nachreagieren und trennt dann 455 Teile organische Phase ab. Der rohe Ester wird gewaschen und destillativ vom Rückstand befreit. Man erhält 431 Teile Destillat mit einem gaschromatographisch ermittelten Vinylmilchsäureisobutylester-Gehalt von 68,2 Gew.% entsprechend einer Ausbeute von 97,2 % d. Th., bezogen auf Vinylmilchsäurenitril in der Gleichgewichtsmischung. Eine fraktionierte Destillation liefert 288 Teile reinen Ester (95 % d. Th.) ; $Kp_{13}$ : 770 °C ; $N_D^{20}$ : 1,427 4.

## Beispiel 4

Wie im Beispiel 3 beschrieben, werden 70 Teile Blausäure mit 174 Teilen Methylvinylketon (96,8 %ig) in 271 Teilen Isobutanol in Gegenwart von 2,7 Teilen Triethylamin zur Reaktion gebracht und in die Cyanhydringleichgewichtsmischung 166 Teilen Chlorwasserstoff eingeleitet, wobei die Temperaturführung zunächst wie beschrieben erfolgt.

Jedoch läßt man die Imidatsuspension noch 2 Stunden bei 40 °C nachreagieren. Nach der üblichen Aufarbeitung erhält man 479 g Primärdestillat mit einem Vinylmilchsäureisobutylestergehalt von 73,4 Gew.% entsprechend einer Ausbeute von 94,4 % d. Th., bez. auf Vinylmilchsäurenitril in der Gleichgewichtsmischung.

## Beispiel 5

Analog Beispiel 3 werden 45 Teile Blausäure mit 105 Teilen Methylvinylketon (99 %) in 166 Teilen Isopropanol in Gegenwart von 1 Teil Triethylamin zur Reaktion gebracht (Cyanhydrinumsatz bei − 40 °C von 92,6 % d. Th.) und die Reaktionsmischung mit 100 Teilen Chlorwasserstoff begast. Anschließend läßt man innerhalb 32 Stunden die Temperatur bis auf + 15 °C ansteigen und arbeitet wie üblich auf. Man erhält 280 Teile Primärdestillat mit einem Gehalt an Vinylmilchsäureisopropylester von 70,5 % entsprechend einer Ausbeute von 90,1 % d. Th., bezogen auf Vinylmilchsäurenitril in der Gleichgewichtsmischung. Reiner Ester $Kp_{70}$ : 92 °C ; $n_D^{20}$ : 1,420 0.

## Beispiel 6

Analog Beispiel 3 werden 46 g Blausäure (99 %ig) mit 120 Teilen 88,6 %igem Methylvinylketon (Wasserazeotrop) in 191 Teilen n-Propanol in Gegenwart von 1 Teil Triethylamin zur Reaktion gebracht (Cyanhydrinumsatz bei − 40 °C von 93 % d. Th.) und in die Reaktionsmischung 110 Teilen Chlorwasserstoff eingeleitet. Anschließend läßt man innerhalb 15 Std. die Temperatur auf + 10 °C ansteigen und arbeitet wie üblich auf. Man erhält 305 Teile Primärdestillat mit einem Gehalt an Vinylmilchsäure-n-propylester von 64,9 Gew.% entsprechend einer Ausbeute von 88,8 % bezogen auf Vinylmilchsäurenitril in der Gleichgewichtsmischung. Reiner Ester : $Kp_{10}$ : 64 °C ; $n_D^{20}$ : 1,429 1.

## Beispiel 7

Analog Beispiel 3 werden 106 Teile Blausäure (99 %ig) mit 263 Teilen Methylvinylketon (99 %ig) in 256 Teilen absolutem Ethanol in Gegenwart von 3,4 Teilen Triethylamin zur Reaktion gebracht (Cyanhydrinumsatz bei − 40 °C von 93 %) und die Reaktionsmischung mit 215 Teilen Chlorwasserstoff begast. Anschließend läßt man die Temperatur innerhalb 10 Stunden auf 20 °C ansteigen und arbeitet wie üblich auf. Zusätzlich wird die wäßrige Phase mit Ether nachextrahiert. Durch fraktionierte Destillation der organischen Phasen lassen sich daraus 477 g reiner Vinylmilchsäureethylester gewinnen. Ausbeute 89,9 % d. Th., bezogen auf Vinylmilchsäurenitril in der Gleichgewichtsmischung. Ohne Nachextraktion der wäßrigen Phase liegt die Ausbeute bei 84,3 %. Reiner Ester : $Kp_{30}$ : 74 °C ; $n_D^{20}$ : 1,426 8.

## Beispiel 8

Analog Beispiel 3 werden 70 Teile Blausäure (98,7 %ig) mit 216 Teilen Methylisopropenylketon (89 %ig) in 205 Teilen abs. Ethanol in Gegenwart von 2 Teilen Triethylamin zur Reaktion gebracht (Cyanhydrinumsatz bei − 40 °C von 88 Mol%) und in die Reaktionsmischung 176 Teilen Chlorwasserstoff eingeleitet. Man läßt 48 Stunden bei Raumtemperatur nachreagieren und hydrolysiert dann mit 695 Teilen 11,8 %iger Natronlauge und trennt nach 5 Stunden das zweiphasige Gemisch. Man erhält 451 g organische Phase, die 370 g Primärdestillat liefert. Der Gehalt an Isopropenylmilchsäureethylester beträgt 73,7 %, entsprechend einer Ausbeute von 86,4 % bezogen auf das Isopropenylmilchsäurenitril in der Gleichgewichtsmischung. Nach der Fraktionierung erhält man einen 97,5 %igen Ester ($n_D^{20}$ : 1,436 0) ; $Kp_{15}$ : 72-73 °C.

## Beispiel 9

In den ersten Kessel einer Mehrstufenkaskade werden pro Std. 49 Teile Blausäure (98,7 %ige), 117

Teile Methylvinylketon (98 %ig), 212 Teile Isobutanol und 1,6 Teile Triethylamin bei – 15 °C gegeben. In 2 nachgeschalteten Reaktionsgefäßen steigt bei – 40 °C der Cyanhydrinumsatz auf 90 Mol%. In den 4. Reaktor werden pro Stunde 110 Teile Chlorwasserstoff bei 0 °C eingeleitet, in den 2 folgenden Reaktoren läßt man das Reaktionsgemisch bei + 15 °C ausreagieren. In den 6. Reaktor werden bei + 35 °C pro Stunde ca. 572 Teile einer 9 %igen wäßrigen Natronlauge so zudosiert, daß ein pH von 3,0 aufrechterhalten werden kann. In einer nachfolgenden Trennflasche wird die organische von der wäßrigen Phase getrennt. Man erhält pro Stunde 381 Teile organische Phase, nach der destillativen Abtrennung des Rückstandes 365 g 67 %igen Vinylmilchsäureisobutylester entsprechend einer Ausbeute von 96,3 % bezogen auf das Vinylmilchsäurenitril in der Cyanhydringleichgewichtsmischung des Kessels 3.

Beispiel 10

In eine Lösung von 22 Teilen HCl-Gas in 28,6 Teilen Ethanol werden 55 Teile Vinylisobutylketoncyanhydrin (90 %ig) innerhalb 1 Stunde bei 0 °C gegeben. Anschließend läßt man 2 Stunden bei + 10 °C und 15 Stunden bei Raumtemperatur nachreagieren, hydrolysiert mit 92,6 g 11 %iger Natronlauge wie beschrieben, wobei jedoch die Nachreaktionszeit bei 30 °C auf 2 Stunden verlängert wird, trennt das zweiphasige Gemisch und arbeitet die organische Phase wie üblich auf. Man erhält 59,7 g Primärdestillat mit einem Gehalt von 86,8 % Vinylisobutylglykolsäureethylester entsprechend einer Ausbeute von 78,5 % d. Th. Reiner Ester : $Kp_{20}$ : 96-98 °C.

**Ansprüche**

1. Verfahren zur Herstellung von Alkenylmilchsäureestern der allgemeinen Formel I

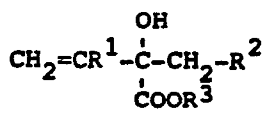 (I)

worin
$R^1$ ein Wasserstoffatom oder eine Methyl- oder Ethylgruppe
$R^2$ ein Wasserstoffatom oder eine Alkylgruppe mit 1-3 Kohlenstoffatomen und
$R^3$ eine Alkylgruppe mit 1-6 Kohlenstoffatomen
darstellen, welches darin besteht, daß man ein Cyanhydrin der allgemeinen Formel II

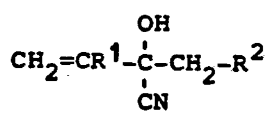 (II)

worin
$R^1$ und $R^2$ dasselbe wie oben bedeuten, mit einem Alkohol der allgemeinen Formel III

$$R^3OH \quad \text{(III)}$$

worin
$R^3$ dasselbe wie oben bedeutet, in Gegenwart von Chlorwasserstoff umsetzt und das so erhaltene Reaktionsgemisch anschließend direkt unter Zugabe von Wasser bei pH 1-6 hydrolysiert.

2. Verfahren gemäß Anspruch 1, dadurch gekennzeichnet, daß man statt des Cyanhydrins eine alkoholische Cyanhydringleichgewichtsmischung aus einem Alkylalkenylketon der allgemeinen Formel IV

$$CH_2 = CR^1\text{—}CO\text{—}CH_2R^2 \quad \text{(IV)}$$

und Blausäure einsetzt.

3. Verfahren nach Anspruch 1 oder 2, dadurch gekennzeichnet, daß man die Umsetzung in einer mehrstufigen Rührkesselkaskade durchführt.

**Claims**

1. A process for the preparation of an alkenyllactic acid ester of the general formula I

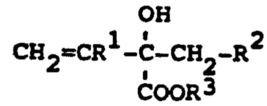

(I)

where

$R^1$ is hydrogen, methyl or ethyl,

$R^2$ is hydrogen or alkyl of 1 to 3 carbon atoms and

$R^3$ is alkyl of 1 to 6 carbon atoms, which consists in reacting a cyanohydrin of the general formula II

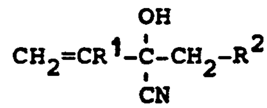

(II)

where

$R^1$ and $R^2$ have the same meanings as above, with an alcohol of the general formula III

$$R^3OH \qquad (III)$$

where

$R^3$ has the same meaning as above, in the presence of hydrogen chloride, and then hydrolyzing the resulting reaction mixture directly with the addition of water at pH 1-6.

2. A process as claimed in claim 1, characterized in that, in place of the cyanohydrin, an equilibrium cyanohydrin mixture, comprising an alkyl alkenyl ketone of the general formula IV

$$CH_2 = CR^1—CO—CH_2R^2 \qquad (IV)$$

and hydrocyanic acid, in alcohol, is employed.

3. A process as claimed in claim 1 or 2, characterized in that the reaction is carried out in a multi-stage stirred kettle cascade.

**Renvendications**

1. Procédé de préparation de lactates d'alcényle de formule I

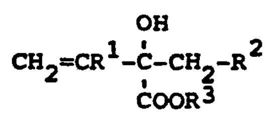

(I)

dans laquelle

$R^1$ représente un atome d'hydrogène ou un groupe méthyle ou éthyle,

$R^2$ un atome d'hydrogène ou un groupe alkyle à 1 à 3 atomes de carbone et

$R^3$ un groupe alkyle à 1 à 6 atomes de carbone, selon lequel on fait réagir, en présence d'acide chlorhydrique une cyanhydrine de formule générale II

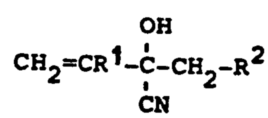

(II)

dans laquelle

$R^1$ et $R^2$ ont la même signification que ci-dessus avec un alcool de formule générale III

$$R^3OH \qquad (III)$$

dans laquelle

$R^3$ a la même signification que ci-dessus, et on hydrolyse ensuite directement le mélange de réaction ainsi obtenu, en ajoutant de l'eau, à un pH de 1 à 6.

2. Procédé selon la revendication 1, caractérisé par le fait qu'au lieu de la cyanhydrine on fait réagir un mélange en équilibre alcoolique de cyanhydrine obtenu à partir d'alkylalcénylcétone de formule générale IV

$$CH_2 = CR^1—CO—CH_2R^2 \qquad (IV)$$

et d'acide cyanhydrique.

3. Procédé selon l'une des revendications 1 ou 2, caractérisé par le fait que l'on effectue la réaction dans une cascade de chaudières à agitateur.